# EUROPEAN PATENT APPLICATION

(11) **EP 1 911 446 A2**
(43) Date of publication of application: **16.04.2008**
(21) Application number: 07115642.6
(22) Date of filing: 12.12.2001
(51) Int. Cl.: A61K 31/00, A61K 31/426, A61K 31/427, A61K 31/4439, A61K 31/506, A61P 25/16, A61P 25/18, A61P 25/24, A61P 25/28

(54) **The use of PPAR-gamma agonists for treating diseases characterised by neuron degeneration, neuron injury or impaired plasticity**

(30) Priority: 18.12.2000 GB 0030845
(62) Divisional of application: 01271216.2
(71) Applicant: SMITHKLINE BEECHAM PLC, Brentford, Middlesex TW8 9GS (GB)
(72) Inventor: Chapman, Gayle, Harlow Essex CM19 5AW (GB); Vinson, Mary, Harlow Essex CM19 5AW (GB)
(74) Representative: Rutter, Keith

(57) **Abstract**

A method for the promotion of growth and/or repair of neurons in diseases or conditions characterised by neuron degeneration , injury or impaired plasticity which method comprises the administration of an effective, non-toxic and pharmaceutically acceptable amount of a PPARγ agonist or a pharmaceutically acceptable derivative thereof.

## Description

This invention relates to a novel treatment and in particular to a method for the promotion of growth and/or repair of neurons in diseases or conditions characterised by neuron degeneration, injury or impaired plasticity.

The process of neurodegeneration is an important factor in the onset and progression of many CNS diseases including stroke, Alzheimer's disease, fronto-temporal dementias (tauopathies), Parkinson's disease, Amyotrophic lateral sclerosis, dementia with Lewy bodies traumatic brain or spinal injury, multiple sclerosis, the spinocerebellar degenerations, multiple systems atrophy, inborn errors of metabolism and Huntington's disease. At present there are no treatments available, which promote axon regeneration following neuronal damage due to such CNS diseases.

In addition increased regeneration of damaged axons and enhanced synaptic plasticity are important mechanisms potentiating functional recovery of the CNS following head and/or spinal cord injury. Such mechanisms may also be beneficial in a number of psychiatric disorders where decreased synaptic plasticity is thought to play a role in the disease pathology.

Currently, therapeutic agents directed towards the treatment of neurodegeneration rely on limiting secondary neuronal damage that result from inflammation following injury and manipulating mechanisms underlying neuronal cell death. In acute diseases such as stroke this approach is limited since rapid medical intervention is required due to the rapidity of cell death following the cessation of blood flow.

Following the onset of stroke, a spontaneous functional recovery is observed in many patients, suggesting that the brain has the ability to repair and/or remodel following injury. Agents that have the potential to enhance this recovery, such as those promoting re-growth and/or repair of neurons may therefore allow intervention to be made much later (potentially days) following the onset of cerebral ischaemia. Therapies that elicit axon sprouting following injury may therefore be valuable in restoring functional synaptic connections lost by the degenerating CNS in chronic and acute neurodegenerative diseases.

Finally, diseases where increased synaptic plasticity may also be beneficial are the psychiatric disorders including schizophrenia and depression. It has been reported that patients undergoing chronic treatment with effective anti-depressants display increased markers of synaptic plasticity. Compounds that enhance the ability of neurons to extend neurites and potentially increase neuroplasticity may therefore be effective in the prophylaxis and treatment of these disorders.

European Patent Application, Publication Number 0306228 discloses certain thiazolidinedione derivatives which are disclosed *inter alia* as having hypoglycaemic and hypolipidaemic activity and activity in treating certain eating disorders. The compound of example 30 of EP 0306228 is 5-(4-[2-(N-methyl-N-(2-pyridyl)amino)ethoxy]benzyl)-2,4-thiazolidinedione (or'Compound (I)').

European Patent Applications, Publication Numbers: 0306228, 0008203, 0139421, 0032128,0428312,0489663,0155845,0257781,0208420,0177353,0319189,0332331, 0332332, 0528734, 0508740; International Patent Application, Publication Numbers 92/18501, 93/02079, 93/22445 and United States Patent Numbers 4687777, 5104888 and 5478852, also disclose certain thiazolidinedione derivatives which are stated to have hypoglycaemic and hypolipidaemic activity.

It is known that the γ-isoform of peroxisome proliferator-activated receptor (herein after PPARγ) is member of a nuclear receptor superfamily that includes receptors for the steroid, thyroid and retinoid hormones (Evans, Science 240, 889-895, (1988)).

It is known from J. Biol. Chem., 270,12963-12966 that thiazolidenediones such as Compound (I) are PPARγ agonists.

Further, PPARγ agonists include non-thiazolidinedione agonists such as the compounds of formula (I) of International application, publication number WO 97/31907 (or EP0888317). A particular compound is 2(S)-(2-benzoyl-phenylamino)-3-{4-[2-5-methyl-2-phenyl-oxazol-4-yl)-ethoxy]-phenyl}-propionic acid (Compound (II)).

It is now surprisingly indicated that PPARγ agonists such as Compound (I) or Compound (II) promote growth and/or repair of neurons and thus are indicated to be effective in the treatment and/or prophylaxis of diseases or conditions characterised by neuron degeneration, injury or impaired plasticity.

Accordingly, the invention provides a method for the promotion of growth and/or repair of neurons in diseases or conditions characterised by neuron degeneration, injury or impaired plasticity which method comprises the administration of an effective, non-toxic and pharmaceutically acceptable amount of a PPARγ agonist, such as Compound (I) or Compound (II), or a pharmaceutically acceptable derivative thereof.

Suitably the method is for the promotion of growth of neurons.

Suitably the method is for the provides a method for the repair of neurons.

Suitable diseases or conditions are those characterised by neuron degeneration.

Suitable diseases or conditions are those characterised by neuron injury.

Suitable diseases or conditions are those characterised by impaired plasticity.

Particular diseases or conditions are characterised by neuron degeneration and thus benefiting from the growth and/or repair of neurons include stroke, Alzheimer's disease, fronto-temporal dementias (tauopathies), Parkinson's disease, Amyotrophic lateral sclerosis, dementia with Lewy bodies, traumatic brain or spinal injury, multiple sclerosis, the spinocerebellar degenerations, multiple systems atrophy, inborn errors of metabolism and Huntington's disease; suitably stroke; suitably Alzheimer's disease; suitably fronto-temporal dementias (tauopathies); suitably Parkinson's disease; suitably Amyotrophic lateral sclerosis; suitably dementia with Lewy bodies; suitably traumatic brain or spinal injury; suitably multiple sclerosis; suitably the spinocerebellar degenerations; suitably multiple systems atrophy; suitably inborn errors of metabolism; or suitably Huntington's disease.

Diseases or conditions characterised by neuron degeneration and/or impaired plasticity include psychiatric disorders such as schizophrenia and depression; suitably schizophrenia; suitably depression.

Particular diseases or conditions characterised by neuronal injury include those conditions associated with head and/or spinal cord injury, including trauma and Multiple Sclerosis; suitably trauma; suitably Multiple Sclerosis.

Suitable PPARγ agonists include thiazolidinediones, especially thiazolidine-2,4-diones, that is a compound comprising a moiety of formula (A):

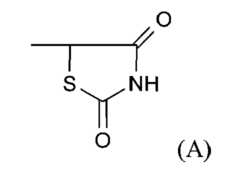

Suitable compounds comprising a moiety of formula (A) include compounds of formula (I): 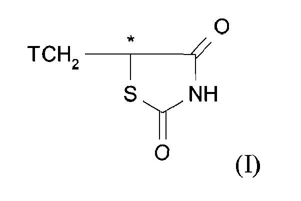 or a tautomeric form thereof and/or a pharmaceutically acceptable salt thereof and/or a pharmaceutically acceptable solvate thereof, wherein T represents an aryl or heterocyclyl group optionally substituted with one or more alkyl groups, aralkyl groups or heterocyclylalkyl groups, the said alkyl, aralkyl and heterocyclylalkyl groups themselves being optionally substituted.

Suitably, the carbon atom marked with an asterisk (*) in formula (I) is a chiral carbon atom.

In particular T represents a moiety selected from the list consisting of (a), (b), (c), (d), (e), (f), (g), (h), and (i):

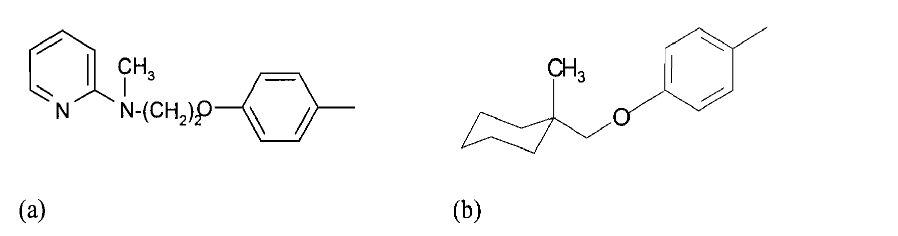

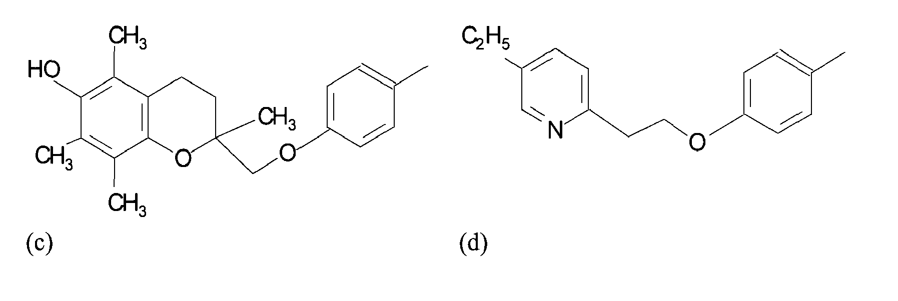

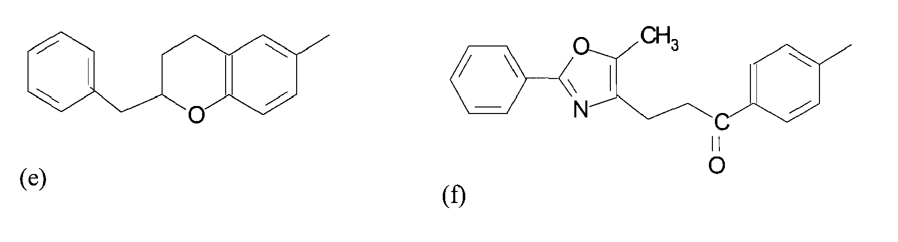

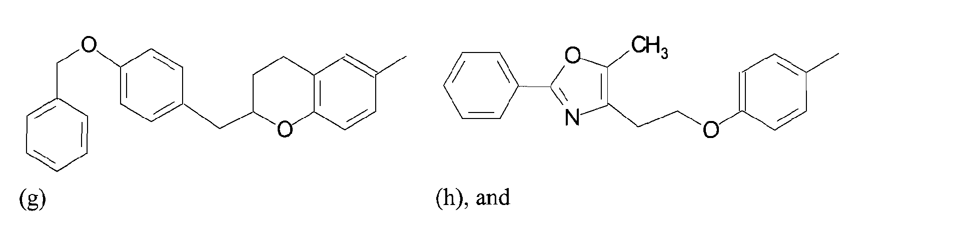

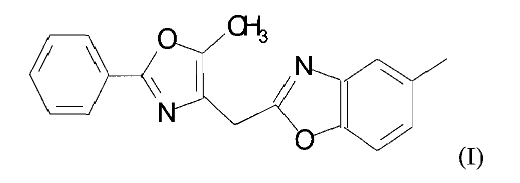

In particular should be mentioned the moieties of formula (a), (b), (c), (d), and (e).

Also included in the treatment of the invention are the PPARγ agonists disclosed in European Patent Applications, Publication Numbers: 0306228, 0008203, 0139421, 0032128, 0428312, 0489663, 0155845, 0257781, 0208420, 0177353, 0319189, 0332331, 0332332, 0528734 and 0508740, International Patent Application, Publication Numbers 92/18501, 93/02079, 93/22445 and United States Patent Numbers 4687777, 5104888 and 5478852, especially the specific example thereof. The contents of these publications are included herein by reference.

Thiazolidinedione PPARγ agonists may exist in one of several tautomeric forms, all of which are encompassed by the present invention as individual tautomeric forms or as mixtures thereof. Where a PPARγ agonist contains a chiral carbon atom, and hence exists in one or more stereoisomeric forms or where one or more geometric isomers exist, it will be appreciated that the method of the present invention encompasses all of the said forms of the PPARγ agonists whether as individual isomers or as mixtures of isomers, including racemates.

Particular examples of thiazolidinediones are those disclosed in EP 0306228 and WO94/05659. Further particular examples are the thiazolidenediones disclosed in EP0139421 and USP 5478852.

A preferred thiazolidinedione is Compound (I).

Further particular thiazolidenediones are, (+)-5-[[4-[(3,4-dihydro-6-hydroxy-2,5,7,8-tetramethyl-2H-1-benzopyran-2-yl)methoxy]phenyl]methyl]-2,4-thiazolidinedione (or troglitazone), 5-[4-[(1-methylcyclohexyl)methoxy]benzyl] thiazolidine-2,4-dione (or ciglitazone), 5-[4-[2-(5-ethylpyridin-2-yl)ethoxy]benzyl] thiazolidine-2,4-dione (or pioglitazone) or 5-[(2-benzyl-2,3-dihydrobenzopyran)-5-ylmethyl)thiazolidine-2,4-dione (or englitazone).

A particular thiazolidinedione is 5-[4-[2-(5-ethylpyridin-2-yl)ethoxy]benzyl] thiazolidine-2,4-dione (or pioglitazone) or a pharmaceutically acceptable derivative thereof such as a hydrochloride salt.

As indicated above, further, suitable PPARγ agonists include non-thiazolidinedione PPARγ agonists such as the compounds of formula (I) of International application, publication number WO 97/31907 (or EP0888317) or a pharmaceutically acceptable derivative thereof. A particular compound of WO 97/31907 (or EP0888317) is Compound (II) or a pharmaceutically acceptable derivative thereof, such as a pharmaceutically acceptable salt or pharmaceutically acceptable solvate thereof.

The contents of the above mentioned publications such as EP 0306228, WO94/05659, WO 97/31907 and EP0888317 are incorporated wholly herein by reference.

When used herein the term 'PPARγ agonist' relates to an agonist, such as a small molecular weight agonist, of the peroxisome proliferator-activated receptor of the gamma subtype, this nuclear receptor is a member of the ligand activated transcription factor family that include the steroid, retinoid and thyroid receptors.

PPARγ agonist activity may be assessed by use of the methodology disclosed by Lehmann et al : Journal of Biological Chem., 270, 12953-12956 (1995).

When used herein the term 'aryl' includes phenyl and naphthyl optionally substituted with up to five, preferably up to three, groups selected from halogen, alkyl, phenyl, alkoxy, haloalkyl, hydroxy, amino, nitro, carboxy, alkoxycarbonyl, alkoxycarbonylalkyl, alkylcarbonyloxy, or alkylcarbonyl groups.

Suitable heterocyclyl groups are aromatic and non-aromatic heterocylic groups.

Suitable non-aromatic heterocylic groups include groups comprising single or fused ring heterocyclic groups comprising up to 4 hetero atoms in each ring selected from oxygen, sulphur or nitrogen, optionally fused to one or more aryl groups.

Suitable aromatic heterocyclyl groups include substituted or unsubstituted, single or fused ring aromatic heterocyclyl groups comprising up to 4 hetero atoms in each ring selected from oxygen, sulphur or nitrogen.

Favoured aromatic heterocyclyl groups include substituted or unsubstituted single ring aromatic heterocyclyl groups having 5 to 7 ring atoms, preferably 5 or 6 ring atoms.

In particular, the aromatic heterocyclyl groups comprise 1, 2 or 3 heteroatoms, especially 1 or 2, selected from oxygen, sulphur or nitrogen.

Suitable substituents for the heterocyclyl include up to 4 substituents selected from the group consisting of: alkyl, alkoxy, aryl and halogen or any two substituents on adjacent carbon atoms, together with the carbon atoms to which they are attached, may form an aryl group, preferably a benzene ring, and wherein the carbon atoms of the aryl group represented by the said two substituents may themselves be substituted or unsubstituted.

It will be appreciated that where the above mentioned definitions of 'aryl', 'heterocyclyl' and the substituents thereof differ from those in the above mentioned patent publications with respect to the particular compounds disclosed therein, that the definitions in the said publications prevail.

When used herein the term 'halogen' refers to fluorine, chlorine, bromine and iodine; preferably chlorine.

When used herein the terms 'alkyl' and 'alkoxy' relate to groups having straight or branched carbon chains, containing up to 12 carbon atoms.

When used herein the term 'acyl' includes alkylcarbonyl groups.

Suitable alkyl groups are C₁₋₁₂ alkyl groups, especially C₁₋₆ alkyl groups e.g. methyl, ethyl, n-propyl, iso-propyl, n-butyl, isobutyl or tert-butyl groups.

Suitable substituents for any alkyl group include those indicated above in relation to the term "aryl".

Suitable derivatives of a PPARγ agonist are pharmaceutically acceptable derivatives, for example salts and solvates.

Suitable derivatives of any particular PPARγ agonist include those disclosed in the above mentioned publications.

Suitable pharmaceutically acceptable salts include salts of salts derived from appropriate acids, such as acid addition salts, or bases.

Suitable pharmaceutically acceptable salts include metal salts, such as for example aluminium, alkali metal salts such as lithium, sodium or potassium, alkaline earth metal salts such as calcium or magnesium and ammonium or substituted ammonium salts, for example those with lower alkylamines such as triethylamine, hydroxy alkylamines such as 2-hydroxyethylamine, bis-(2-hydroxyethyl)-amine or tri-(2-hydroxyethyl)-amine, cycloalkylamines such as bicyclohexylamine, or with procaine, dibenzylpiperidine, N-benzyl-b-phenethylamine, dehydroabietylamine, N,N'-bisdehydroabietylamine, glucamine, N-methylglucamine or bases of the pyridine type such as pyridine, collidine, quinine or quinoline.

Suitable acid addition salts include pharmaceutically acceptable inorganic salts such as the sulphate, nitrate, phosphate, borate, hydrochloride and hydrobromide and pharmaceutically acceptable organic acid addition salts such as acetate, tartrate, maleate, citrate, succinate, benzoate, ascorbate, methane-sulphonate, a-keto glutarate and a-glycerophosphate, especially the maleate salt.

Suitable pharmaceutically acceptable salts of Compound (I) are as disclosed in EP 0306228 and WO94/05659 and include maleate salts.

Suitable pharmaceutically acceptable solvates include hydrates.

Suitable pharmaceutically acceptable solvates of Compound (I) are as disclosed in EP 0306228 and WO94/05659 and include hydrates.

Suitable pharmaceutically acceptable derivatives, such as salts or solvates, of Compound (II) are as disclosed in WO 97/31907 (or EP0888317).

The PPARγ agonists, such as the thiazolidinediones or non-thiazolidinediones such as the compounds disclosed in WO 97/31907 (or EP0888317), referred to herein are conveniently prepared according to the methods disclosed in the above mentioned patent publications in which they are disclosed: Thus Compound (I), or the tautomeric form thereof, and/or a pharmaceutically acceptable salt thereof, and/or a pharmaceutically acceptable solvate thereof, may be prepared using the processes described in EP 0306228 and WO94/05659. Also Compound (II), or a pharmaceutically acceptable derivative thereof, such as salts or solvates thereof, may be prepared using the processes described in WO 97/31907 (or EP0888317).

The salts and/or solvates of the thiazolidinediones may be prepared and isolated according to conventional procedures for example those disclosed in the, above mentioned, patent publications.

The present invention also provides a PPARγ agonist or a pharmaceutically acceptable derivative thereof, for use in the promotion of growth and/or repair of neurons in diseases or conditions characterised by neuron degeneration, injury or impaired plasticity.

The present invention also provides a PPARγ agonist or a pharmaceutically acceptable derivative thereof, for use in the manufacture of a medicament for the promotion of growth and/or repair of neurons in diseases or conditions characterised by neuron degeneration, injury or impaired plasticity.

In the above mentioned method the PPARγ agonist, may be administered per se or, preferably, as a pharmaceutical composition also comprising a pharmaceutically acceptable carrier.

In the treatment of the invention, the PPARγ agonist mentioned herein is formulated and administered in accordance with the methods disclosed in the above mentioned patent applications and patents.

Accordingly, the present invention also provides a pharmaceutical composition for the promotion of growth and/or repair of neurons in diseases or conditions characterised by neuron degeneration, injury or impaired plasticity, which composition comprises a PPARγ agonist, or a pharmaceutically acceptable derivative thereof, and a pharmaceutically acceptable carrier therefor.

As used herein the term 'pharmaceutically acceptable' embraces compounds, compositions and ingredients for both human and veterinary use: for example the term 'pharmaceutically acceptable salt' embraces a veterinarily acceptable salt.

The active compounds are usually administered as the sole medicament agent but they may be administered in combination with other medicament agents.

The said combination also comprises co-administration of a PPARγ agonist, such as Compound (I) or Compound (II) or a pharmaceutically acceptable derivative thereof, or a compound of formula (I), or a pharmaceutically acceptable derivative thereof, and an additional medicament agent or the sequential administration of a PPARγ agonist, such as Compound (I) or Compound (II) or a pharmaceutically acceptable derivative thereof, or a compound of formula (I), or a pharmaceutically acceptable derivative thereof, and the additional medicament agent.

Co-administration includes administration of a pharmaceutical composition which contains both a PPARγ agonist, such as Compound (I) or Compound (II) or a pharmaceutically acceptable derivative thereof, or a compound of formula (I), or a pharmaceutically acceptable derivative thereof, and the additional medicament agent or the essentially simultaneous administration of separate pharmaceutical compositions of a compound of formula (I), or a pharmaceutically acceptable derivative thereof, and the additional medicament agent.

The composition may, if desired, be in the form of a pack accompanied by written or printed instructions for use.

Usually the pharmaceutical compositions of the present invention will be adapted for oral administration, although compositions for administration by other routes, such as by injection and percutaneous absorption are also envisaged.

Particularly suitable compositions for oral administration are unit dosage forms such as tablets and capsules. Other fixed unit dosage forms, such as powders presented in sachets, may also be used.

In accordance with conventional pharmaceutical practice the carrier may comprise a diluent, filler, disintegrant, wetting agent, lubricant, colourant, flavourant or other conventional adjuvant.

Typical carriers include, for example, microcrystalline cellulose, starch, sodium starch glycollate, polyvinylpyrrolidone, polyvinylpolypyrrolidone, magnesium stearate, sodium lauryl sulphate or sucrose.

Suitable dosages of the PPARγ agonist include the known doses for these compounds as described or referred to in reference texts such as the British and US Pharmacopoeias, Remington's Pharmaceutical Sciences (Mack Publishing Co.), Martindale The Complete Drug Reference (London, The Pharmaceutical Press, 32nd Edition) or the above mentioned publications or doses which can be determined by standard procedures.

Suitable dosages of the Compound (I) include those disclosed in EP 0306228 and WO94/05659 and 1, 2, 3, 4, 5, 6, 7, 8, 9, 10, 11 or 12 mg of Compound (I).

Particular dosages of Compound (I) are 2mg, 4mg and 8mg.

Suitable doses of Compound (II), or a pharmaceutically acceptable derivative thereof, such as salts or solvates thereof, are as disclosed in WO 97/31907 (or EP0888317).

The composition of the invention may be administered from 1 to 6 times a day, but most preferably 1 or 2 times per day, or some other such period as disclosed in the above mentioned publications.

The solid oral compositions may be prepared by conventional methods of blending, filling or tabletting. Repeated blending operations may be used to distribute the active agent throughout those compositions employing large quantities of fillers. Such operations are of course conventional in the art. The tablets may be coated according to methods well known in normal pharmaceutical practice, in particular with an enteric coating.

Oral liquid preparations may be in the form of, for example, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia; non-aqueous vehicles (which may include edible oils), for example almond oil, fractionated coconut oil, oily esters such as esters of glycerine, propylene glycol, or ethyl alcohol; preservatives, for example methyl or propyl p-hydroxybenzoate or sorbic acid; and if desired conventional flavouring or colouring agents.

For parenteral administration, fluid unit dosage forms are prepared utilizing the compound and a sterile vehicle, and, depending on the concentration used, can be either suspended or dissolved in the vehicle. In preparing solutions the compound can be dissolved in water for injection and filter sterilized before filling into a suitable vial or ampoule and sealing. Advantageously, adjuvants such as a local anaesthetic, a preservative and buffering agents can be dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

Compositions may contain from 0.1% to 99% by weight, preferably from 10-60% by weight, of the active material, depending upon the method of administration.

Compositions may, if desired, be in the form of a pack accompanied by written or printed instructions for use.

The compositions are formulated according to conventional methods, such as those disclosed in standard reference texts, for example the British and US Pharmacopoeias, Remington's Pharmaceutical Sciences (Mack Publishing Co.), Martindale The Complete Drug Reference (London, The Pharmaceutical Press, 32nd Edition) and Harry's Cosmeticology (Leonard Hill Books).

One index of synaptic plasticity is increased synaptic transmission. This can be measured in cultured hippocampal neurons using electrophysiological recordings as described by Levine E S, Crozier R A, Black I B, Plummer M R. "Brain derived neurotrophic factor modulates hippocampal synaptic transmission by increasing N-methyl-D aspartic acid receptor activity", in Proc. Natl. Acad. Sci USA Vol 95 pp10235-10239 (1998). Thus the neurons would be treated with test compound, such as Compound (I), and then their synaptic transmission determined against a control following glutamate exposure.

No adverse toxicological effects are expected for the compositions or methods of the invention in the above mentioned dosage ranges.

The following descriptions and examples illustrate the invention but do not limit it in any way.

### Description and Examples

### Primary neuronal cell culture

The hippocampi of gestational day 18 rat embryos were dissected out, incubated in trypsin (0.08%, 30min at 37 °C) and dissociated mechanically (Skaper et al., 1990). Hippocampal cells were resuspended in neurobasal medium supplemented with B27, anti-oxidants, 1mM glutamine, 25 µM glutamate, 1 mM pyruvate, +/- Compound 1 (10nM-5µM) or vehicle (DMSO) control.

For outgrowth assays, cells were plated at a density of 3000 cells/well into 96 well dishes that had previously been coated with poly-D-lysine followed by 10% FCS. For RNA isolation, cells were plated at 1x10⁶ cells/well into a 35mm tissue culture dish that had previously been coated with poly-D-lysine followed by 10% FCS.

### RNA isolation and reverse transcription

RNA was prepared from cells lysed in Tri- reagent (Sigma, Dorset, UK) using 1 ml of Tri-reagent per 10 cm² plate. Total RNA was extracted from the tissue according to the manufacturer's suggested protocol with the addition of an extra chloroform extraction step and phase separation and an extra wash of the isolated RNA in 70% ethanol. The RNA was resuspended in autoclaved, double distilled water and the concentration calculated by A₂₆₀ measurement. RNA quality was assessed by electrophoresis on a 1% agarose gel. First strand cDNA was synthesised using oligo(dT)₁₅ and 500ng of each RNA sample; 0.01 M dithiothreitol, 0.5 mM each dNTP, 0.5 µg oligo(dT)₁₅ primer, 40 U RNAseOUT ribonuclease inhibitor (Life Technologies, Paisley, UK), 200 U SuperscriptII reverse transcriptase (Life Technologies, Paisley, UK). Reverse transcription reactions were performed in duplicate along with an additional reaction in which the reverse transcriptase enzyme was omitted to allow for assessment of genomic DNA contamination of the RNA. Taqman PCR was carried out using an ABI prism 7700 sequence detector (Perkin Elmer, Cheshire, UK) under the following conditions; 50°C for 2 minutes, 95°C for 10 minutes followed by forty cycles of 95°C for 15 seconds, 60°C for 1 minute. The reaction mixture contained cDNA samples (5µl of 20 µl RT reaction); 2.5 mM MgCl₂, 0.2 mM dATP, dCTP, dGTP and dUTP, 0.1 µM each primer, 0.05 µM Taqman probe, 0.01 U AmpErase uracil-N-glycosylase (Perkin Elmer, Cheshire, UK), 0.0125 U Amplitaq Gold DNA polymerase (Perkin Elmer, Cheshire, UK). Taqman primer and probe set for rat PPAR-gamma were designed using Primer Express software (Perkin Elmer, Cheshire, UK). Primer and probe sequences (5' - 3') (forward primer, reverse primer, Taqman probe);
Forward primer: CTGACCCAATGGTTGCTGATTAC
Reverse primer: GGACGCAGGCTCTACTTTGATC
Probe: FAM-AAATATGACCTGAAGCTCCAAGAATACCAAAGTGC-TAMRA

(Amplicon is 80 bp and the amplicon coordinates within the rat PPARgamma mRNA (accession AB011365) are 176-255)

Triplicate amplifications were carried out, following amplification, a representitive amplicon from each sample (2µl) was electrophoressed on a 4% agarose gel to determine molecular weight.

### Neurite outgrowth assays

Compound (I) was solubilised in DMSO and added to culture medium at time of cell plating at a dilution of 1:1000. Vehicle only (1:1000) was added to culture medium of untreated controls. After 48 hours, cells were fixed with 4% paraformaldehyde for 1 hour on ice, washed with PBS and stained using Coomassie. Assays were quantified using a KS300 image analysis system (Imaging Associates, UK). For each cell measured, the length from the edge of the cell to the end of the longest neurite was measured for 100 cells/well for each treatment in triplicate. All data are means and SEM pooled from three independent experiments#. Results are expressed as a percentage of the length of neurites of cells treated with vehicle alone.

# Compound (I) at 0.01 and 0.05µM has been analysed in one experiment only (n=3)

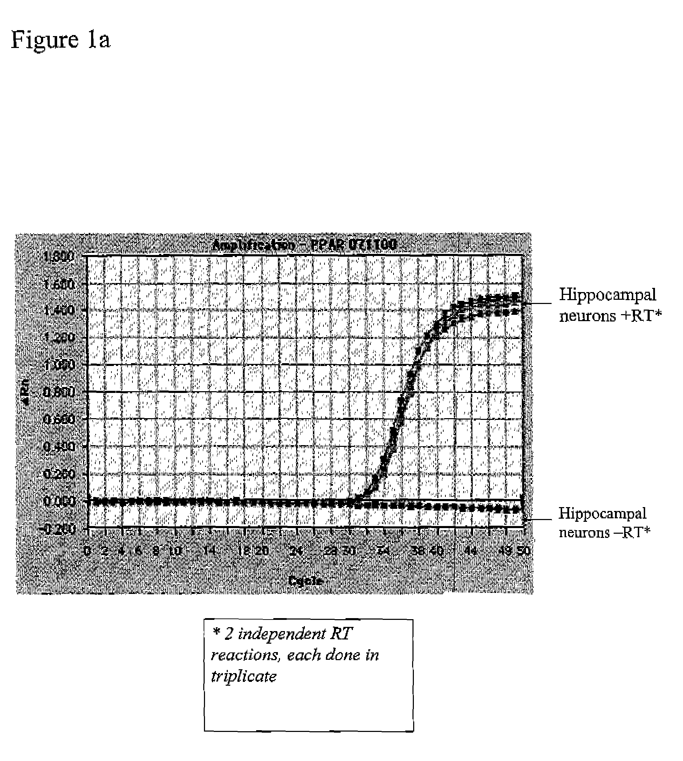

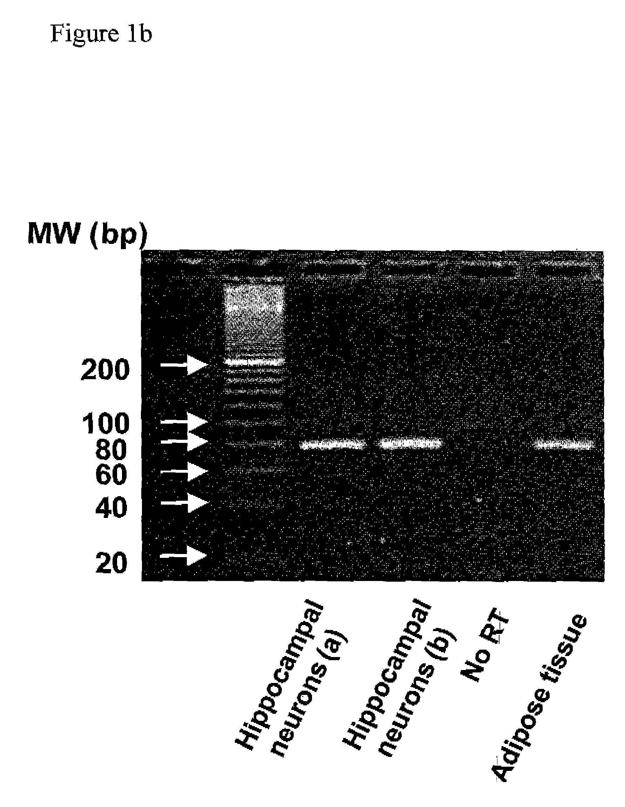

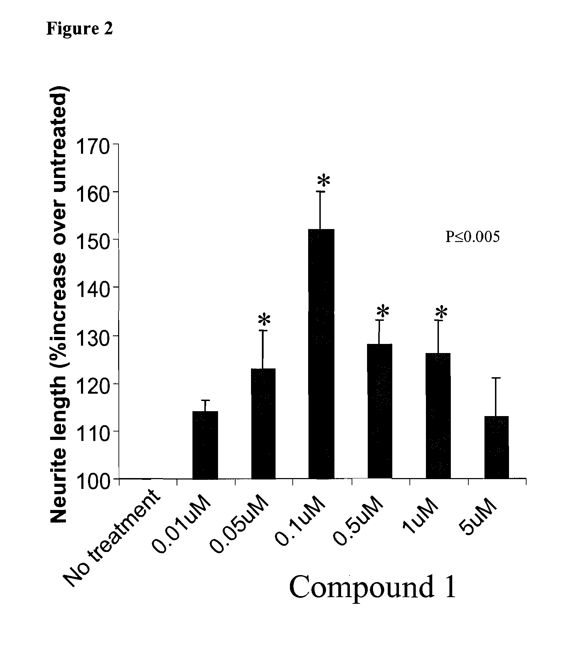

**Results:** the results are expressed in the attached Figures 1(a), 1(b) and 2, which are as follows:
**Figure 1(a):** *PPAR-gamma is expressed by rat primary hippocampal neurons.* (a) Real-time PCR (Taqman) amplification plot of PPAR-gamma in cDNA derived from rat primary hippocampal neurons;
**Figure 1(b)**: Amplicons from taqman analysis separated on 4% agarose gel. cDNA derived from adipose used as a positive control. Molecular weight as predicted; and
**Figure 2:** Compound (I) increases neurite outgrowth in rat primary hippocampal neurons in a dose dependant manner. Compound (I) at 100nM increases outgrowth over untreated controls by approx. 50%.

### References

Skaper SD, Facci L, Milani D, Leon A, and Toffano G (1990). In Methods in Neurosciences, Vol 2, Academic Press, San Diego.

## Claims

1. A method for the promotion of growth and/or repair of neurons in diseases or conditions **characterised by** neuron degeneration, injury or impaired plasticity which method comprises the administration of an effective, non-toxic and pharmaceutically acceptable amount of a PPARγ agonist or a pharmaceutically acceptable derivative thereof.

2. A method according to claim 1, wherein the diseases or conditions are **characterised by** neuron degeneration.

3. A method according to claim 2, wherein the diseases or conditions are selected from the list consisting of: stroke, Alzheimer's disease, fronto-temporal dementias (tauopathies), Parkinson's disease, Amyotrophic lateral sclerosis, dementia with Lewy bodies, traumatic brain or spinal injury, multiple sclerosis, the spinocerebellar degenerations, multiple systems atrophy, inborn errors of metabolism and Huntington's disease

4. A method according to claim 1, wherein the diseases or conditions are
**characterised by** neuron degeneration and/or impaired plasticity.

5. A method according to claim 4, wherein the diseases or conditions are selected from schizophrenia and depression.

6. A method according to claim 1, wherein the diseases or conditions are
**characterised by** neuronal injury.

7. A method according to claim 6, wherein the diseases or conditions are furhter
**characterised by** head and/or spinal cord injury.

8. A method according to claim 7, wherein the diseases or conditions are selected from trauma and Multiple Sclerosis.

9. A method according to claim 1, wherein the PPARγ agonist is a compound comprising a moiety of formula (A):

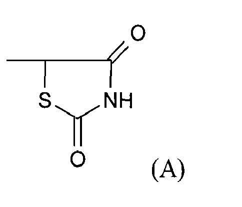

10. A method according to claim 1, wherien the PPARγ agonist is selected from the list consisting of: is 5-(4-[2-(N-methyl-N-(2-pyridyl)amino)ethoxy]benzyl)-2,4-thiazolidinedione, (+)-5-[[4-[(3,4-dihydro-6-hydroxy-2,5,7,8-tetramethyl-2H-1-benzopyran-2-yl)methoxy]phenyl]methyl]-2,4-thiazolidinedione (or troglitazone), 5-[4-[(1-methylcyclohexyl)methoxy]benzyl] thiazolidine-2,4-dione (or ciglitazone), 5-[4-[2-(5-ethylpyridin-2-yl)ethoxy]benzyl] thiazolidine-2,4-dione (or pioglitazone) and 5-[(2-benzyl-2,3-dihydrobenzopyran)-5-ylmethyl)thiazolidine-2,4-dione (or englitazone).

11. A method according to claim 1, wherein the PPARγ agonist is a non-thiazolidinedione PPARγ agonist.

12. A method according to claim 1, wherein the PPARγ agonist is 2(S)-(2-benzoylphenylamino)-3-{4-[2-5-methyl-2-phenyl-oxazol-4-yl)-ethoxy]-phenyl}-propionic acid or a pharmaceitically acceptable derivative thereof.

13. A PPARγ agonist or a pharmaceutically acceptable derivative thereof, for use in the promotion of growth and/or repair of neurons in diseases or conditions **characterised by** neuron degeneration, injury or impaired plasticity.

14. A PPARγ agonist or a pharmaceutically acceptable derivative thereof, for use in the manufacture of a medicament for the promotion of growth and/or repair of neurons in diseases or conditions **characterised by** neuron degeneration, injury or impaired plasticity.

15. A pharmaceutical composition for use in the promotion of growth and/or repair of neurons in diseases or conditions **characterised by** neuron degeneration, injury or impaired plasticity, which composition comprises a PPARγ agonist, or a pharmaceutically acceptable derivative thereof, and a pharmaceutically acceptable carrier therefor.
